# EUROPEAN PATENT SPECIFICATION

(11) **EP 4 103 269 B1**
(45) Date of publication and mention of the grant of the patent: **10.12.2025**
(21) Application number: 21710699.6
(22) Date of filing: 11.02.2021
(51) Int. Cl.: A61N 1/375, A61N 1/378, H01M 10/00, A61B 5/00

(54) **HERMETICALLY SEALED IMPLANTABLE MEDICAL DEVICE AND METHOD OF FORMATION**
HERMETISCH DICHTES IMPLANTIERBARES MEDIZINPRODUKT UND HERSTELLUNGSVERFAHREN
DISPOSITIF MÉDICAL IMPLANTABLE HERMÉTIQUEMENT SCELLÉ ET MÉTHODE DE FORMATION

(30) Priority: 13.02.2020 US 202062976079 P
(43) Date of publication of application: 21.12.2022
(73) Proprietor: Cardiac Pacemakers, Inc., St. Paul, Minnesota 55112 (US)
(72) Inventor: ENGLISH, James M., Cahir (IE); BOBGAN, Jean M., Maple Grove, Minnesota 55311 (US); MAILE, Keith R., New Brighton, Minnesota 55112 (US); BALCZEWSKI, Ron A., Bloomington, Minnesota 55437 (US)
(74) Representative: Pfenning, Meinig & Partner mbB
(86) International application number: PCT/US2021/017610
(87) International publication number: WO 2021/163291

(56) References cited:
- US-A1- 2011 190 842
- US-A1- 2012 303 105
- US-A1- 2014 214 133
- US-A1- 2017 303 424
- US-A1- 2018 272 134

## Description

### TECHNICAL FIELD

Embodiments of the present disclosure relate to medical devices and systems for sensing physiological parameters and/or delivering therapy. More specifically, embodiments of the disclosure relate to devices and methods for a hermetically sealed implantable medical device.

### BACKGROUND

Implantable medical devices (IMDs) may be configured to sense physiological parameters and/or provide therapy and may include one or more electrodes for performing aspects of these functions. IMDs may also include antennas for communicating with other devices. Conventionally, devices such as programmers have been used to cause IMDs to take various actions such as for example, marking recordings of physiological parameters, initiating communications with other devices, and the like. Documents US 2011/190842, US 2017/303424, US 2018/272134 and US 2012/303105 disclose different known implantable devices.

### SUMMARY OF THE DISCLOSURE

Exemplary embodiments of the present disclosure include, but are not limited to, the following examples.

In an example, an implantable medical device (IMD) configured to sense one or more physiological parameters of a subject is provided, the IMD comprising: a power source; a housing enclosing the power source, the housing comprising a first side, a second side, a first end, and a second end, wherein the first side is opposite the second side and the first end is opposite the second end, and wherein a first distance between the first and second ends is greater than a second distance the first and second sides; a printed circuit board arranged on the first side of the housing and conductively coupled to the power source; a non-conductive enclosure arranged over the printed circuit board and hermetically sealing the printed circuit board, the non-conductive enclosure comprising an outer surface; and first and second electrodes arranged on the outer surface of the non-conductive enclosure, wherein the first external electrode is coupled to the printed circuit board by a first trace and the second external electrode is coupled to the printed circuit board by a second trace.

In an example of said device, the non-conductive enclosure is formed from a liquid crystal polymer or an epoxy.

In an example of said device, the connector traces are secured in place on the housing by one of more frames.

In example of said device, the frames are composed of non-conductive material and create boundaries to limit the movement of the traces.

In an example of said device, the device further comprises an antenna arranged within or on the non-conductive enclosure and coupled to the circuit board.

In an example of said device, the antenna comprises a first portion arranged parallel to the longitudinal axis.

In an example of said device, the antenna further comprises a second portion arranged perpendicular to the longitudinal axis.

In an example of said device, the outer surface of the IMD comprises an external seal configured to hermetically seal the IMD from the surroundings.

In an example of said device, the external seal is an atomic deposit layer.

In an example of said device, the housing is comprised of a metallic material.

In an example of said device, a third electrode and a fourth electrode are arranged on an outer surface of the housing.

In an example, the present disclosure provides a method of forming a hermetically-sealed implantable device is provided, comprising: arranging a circuit board subassembly onto a housing enclosing a power source; arranging first segments of connector traces along the housing and arranging second segments of the connector traces to project from the housing, wherein first ends of the connector traces connects to the circuit board subassembly; arranging one or more frames over the first segments of the connector traces to hold the first segments of the connector traces in place; forming a non-conductive enclosure over the subassembly, to create an encasing that leaves portions of the second segments of the connector traces exposed above the outer surface of the non-conductive enclosure; removing the portions of the second segments of the connector traces exposed above the outer surface; and arranging two electrodes on the outer surface of the non-conductive enclosure and connecting the two electrodes to the connector traces.

In an example of said method, the electrodes are arranged on other surface of the non-conductive enclosure through one of the techniques of a laser weld, deposit, sputter, or spray/ink jet method.

In an example of said method, a hermetic seal is applied to the implantable device, after arranging the electrodes on the outer surface.

In an example, said method further comprises forming an antenna within or on the non-conductive enclosure.

In an example according to the present disclosure, an implantable medical device (IMD) configured to sense one or more physiological parameters of a subject is provided, the IMD comprising; a power source; a housing enclosing the power source, the housing comprising a first side and a second side extending along a longitudinal axis between a first end and a second end, wherein the first side is opposite the second side and the first end is opposite the second end, and wherein a first distance between the first and second ends is greater than a second distance the first and second sides; a printed circuit board arranged on the first side of the base and conductively coupled to the power source; a non-conductive enclosure arranged over the printed circuit board and hermetically sealing the printed circuit board, the non-conductive enclosure comprising an outer surface; and first and second electrodes arranged on the outer surface of the non-conductive enclosure, wherein the first external electrode is coupled to the printed circuit board by a first trace and the second external electrode is coupled to the printed circuit board by a second trace.

In an example of said IMD, the non-conductive enclosure is formed from a liquid crystal polymer or an epoxy.

In an example of said IMD, the connector traces are secured in place on the housing by one of more frames.

In example of said IMD, the frames are composed of non-conductive material and create boundaries to limit the movement of the traces.

In an example of said IMD, the device further comprises an antenna arranged within or on the non-conductive enclosure and coupled to the circuit board.

In an example of said IMD, the antenna comprises a first portion arranged parallel to the longitudinal axis.

In an example of said IMD, the antenna further comprises a second portion arranged perpendicular to the longitudinal axis.

In an example of said IMD, the outer surface of the IMD comprises an external seal configured to hermetically seal the IMD from the surroundings.

In an example of said IMD, the external seal is an atomic deposit layer.

In an example of said IMD, the housing is comprised of a metallic material.

In an example of said IMD, a third electrode and a fourth electrode are arranged on an outer surface of the housing.

In an example according to the present disclosure, a method of forming a hermetically-sealed implantable device is provided, said method comprising: arranging a circuit board subassembly onto a hosing enclosing a power source; arranging first segments of connector traces along the housing and arranging second segments of the connector traces to project from the housing, wherein first ends of the connector traces connects to the circuit board subassembly; arranging one or more frames over the first segments of the connector traces to hold the first segments of the connector traces in place; forming the non-conductive enclosure over the subassembly, to create an encasing that leaves portions of the second segments of the connector traces exposed above the outer surface of the non-conductive enclosure; removing portions of the second segments of the connector traces exposed above the outer surface; and arranging two electrodes on the outer surface of the non-conductive enclosure and connecting the two electrodes to the connector traces.

In an example, said method further comprises the first segments of the connector traces are attached to the printed circuit board through the technique of soldering the components together.

In an an example of said method, the electrodes are arranged on other surface of the non-conductive enclosure through one of the techniques of a laser weld, deposit, sputter, or spray/ink jet method.

In an an example of said method, a hermetic seal is applied to the implantable device, after arranging the electrodes on the outer surface.

In an an example of said method, the hermetic seal applied is an atomic layer deposit.

In a further example according to the present description, a hermetically-sealed implantable medical device (IMD) is provided, the IMD comprising: a power source; a housing enclosing the power source; a printed circuit board arranged on the first side of the housing and conductively coupled to the power source; a non-conductive enclosure arranged over the printed circuit board and hermetically sealing the printed circuit board, the non-conductive enclosure comprising an outer surface; and at least one electrode arranged on the outer surface of the non-conductive enclosure.

In an example, said device further comprises an antenna arranged within or on the non-conductive enclosure and connected to the printed circuit board.

In an example of said device, the antenna further comprises a first portion arranged parallel to the longitudinal axis between the first end and the second end of the power source and a second portion arranged perpendicular to the longitudinal axis.

In an example of said device, the at least one electrode comprises a plurality of electrodes.

While multiple embodiments are disclosed, still other embodiments of the present disclosure will become apparent to those skilled in the art from the following detailed description, which shows and describes illustrative embodiments of the subject matter disclosed herein. Accordingly, the drawings and detailed description are to be regarded as illustrative in nature and not restrictive.

### BRIEF DESCRIPTION OF THE DRAWINGS

FIG. 1 is a schematic illustration of a system having an implantable medical device (IMD) and a receiving device, in accordance with embodiments of the present disclosure.
FIG. 2 is a perspective view of a hermetically sealed IMD, in accordance with embodiments of the present disclosure.
FIG. 3A is a perspective view of a portion of a sealed IMD, in accordance with embodiments of the present disclosure.
FIG. 3B is a perspective view of a printed circuit board and power source subassembly with connector traces and guiding frames, in accordance with embodiments of the present disclosure.
FIG. 4 is a schematic diagram of an electrical subassembly, in accordance with embodiments of the present disclosure.
FIG. 5 is a schematic diagram of the IMD and the electrical subassembly in operation with a receiving device, in accordance with embodiments of the present disclosure.
FIG. 6 is a front-facing view of a printed circuit board and power source subassembly, in accordance with embodiments of the present disclosure.
FIG. 7 is a flow chart of a method of forming a hermetically sealed IMD, in accordance with embodiments of the present disclosure.

While the subject matter disclosed herein is amenable to various modifications and alternative forms, specific embodiments have been shown by way of example in the drawings and are described in detail below. The intention, however, is not to limit the disclosure to the particular embodiments described. On the contrary, the disclosure is intended to cover all modifications, equivalents, and alternatives falling within the scope of the subject matter disclosed herein. The present invention is set out in the appended claims.

Although the term "block" may be used herein to connote different elements illustratively employed, the term should not be interpreted as implying any requirement of, or particular order among or between, various steps disclosed herein unless and except when explicitly referring to the order of individual steps.

### DETAILED DESCRIPTION

The size of an implantable medical device (IMD) is constrained due to being implanted in a patient. Due to these constraints, the power supply of the IMD can be a limiting factor in how much functionality can be incorporated into the IMD. Therefore, transmitting sensor measurements to an external device can be useful for processing the sensor measurements. IMDs often include a header made of a non-conductive material to transmit the sensor measurements to the external device. However, including a header may reduce the size of the power supply that can be included in an IMD. The embodiments disclosed herein provide a solution to this problem.

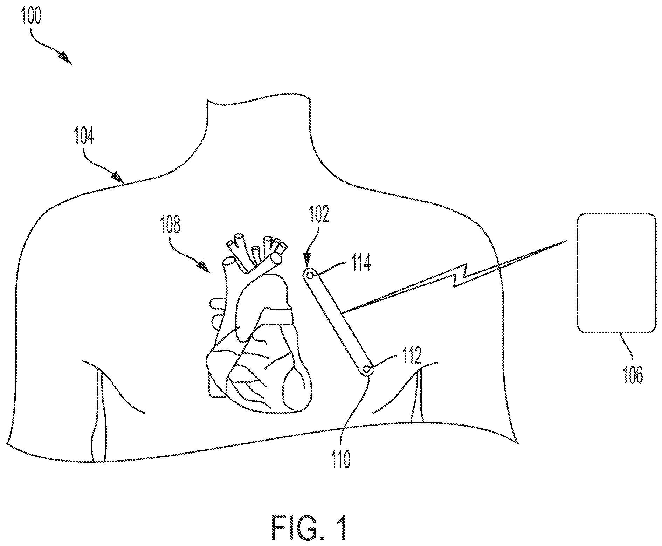

FIG. 1 is a schematic illustration of a system 100 including an IMD 102 implanted within a patient's body 104 and configured to communicate with a receiving device 106. In embodiments, the IMD 102 may be implanted subcutaneously within an implantation location or pocket in the patient's chest or abdomen and may be configured to monitor (e.g., sense and/or record) physiological parameters associated with the patient's heart 108. In embodiments, the IMD 102 may be an implantable cardiac monitor (ICM) (e.g., an implantable diagnostic monitor (IDM), an implantable loop recorder (ILR), etc.) configured to record physiological parameters such as, for example, one or more cardiac activation signals, heart sounds, blood pressure measurements, oxygen saturations, and/or the like.

In certain instances, the IMD 102 may be configured to monitor physiological parameters that may include one or more signals indicative of a patient's physical activity level and/or metabolic level, such as an acceleration signal. In certain instances, the IMD 102 may be configured to monitor physiological parameters associated with one or more other organs, systems, and/or the like. The IMD 102 may be configured to sense and/or record at regular intervals, continuously, and/or in response to a detected event. In certain instances, such a detected event may be detected by one or more sensors of the IMD 102, another IMD (not shown), an external device (e.g., the receiving device 106), and/or the like. In addition, the IMD 102 may be configured to detect a variety of physiological signals that may be used in connection with various diagnostic, therapeutic, and/or monitoring implementations. For example, the IMD 102 may include sensors or circuitry for detecting respiratory system signals, cardiac system signals, and/or signals related to patient activity. In certain instances, the IMD 102 may be configured to sense intrathoracic impedance, from which various respiratory parameters may be derived, including, for example, respiratory tidal volume and minute ventilation. Sensors and associated circuitry may be incorporated in connection with the IMD 102 for detecting one or more body movement or body posture and/or position related signals. For example, accelerometers and/or GPS devices may be employed to detect patient activity, patient location, body orientation, and/or torso position.

For purposes of illustration, and not of limitation, various embodiments of devices that may be used to record physiological parameters in accordance with the present disclosure are described herein in the context of IMDs that may be implanted under the skin in the chest region of a patient.

As shown, the IMD 102 may include a housing 110 having two electrodes 112 and 114 integrated into and/or coupled thereto. According to certain instances, the IMD 102 may include any number of electrodes (and/or other types of sensors such as, e.g., thermometers, barometers, pressure sensors, optical sensors, motion sensors, and/or the like) in any number of various types of configurations, and the housing 110 may include any number of different shapes, sizes, and/or features. In certain instances, the IMD 102 may be configured to sense physiological parameters and record the physiological parameters. For example, the IMD 102 may be configured to activate (e.g., periodically, continuously, upon detection of an event, and/or the like), record (e.g., physiological parameters) in a memory, and communicate that recorded data to a receiving device 106. In the housing of an IMD 102, for example, the IMD 102 may activate, record cardiac signals for a certain period of time, deactivate, and/or activate to communicate the recorded signals to the receiving device 106.

In various instances, the receiving device 106 may be, for example, a programmer, controller, patient monitoring system, and/or the like. Although illustrated in FIG. 1 as an external device, the receiving device 106 may include an implantable device configured to communicate with the IMD 102 that may, for example, be a control device, another monitoring device, a pacemaker, an implantable defibrillator, a cardiac resynchronization therapy (CRT) device, and/or the like, and may be an implantable medical device known in the art or later developed, for providing therapy and/or diagnostic data about the patient and/or the IMD 102. In certain instances, the IMD 102 may be a pacemaker, an implantable cardioverter defibrillator (ICD) device, or a cardiac resynchronization therapy (CRT) device. In certain instances, the IMD 102 may include both defibrillation and pacing/CRT capabilities (e.g., a CRT-D device).

The system 100 may be used to implement coordinated patient measuring and/or monitoring, diagnosis, and/or therapy in accordance with embodiments of the disclosure. The system 100 may include, for example, one or more patient-internal medical devices, such as an IMD 102, and one or more patient-external medical devices, such as receiving device 106. The receiving device 106 may be configured to perform monitoring, and/or diagnosis and/or therapy functions external to the patient (i.e., not invasively implanted within the patient's body). The receiving device 106 may be positioned on the patient, near the patient, or in any location external to the patient.

The IMD 102 and the receiving device 106 may communicate through a wireless link. For example, the IMD 102 and the receiving device 106 may be coupled through a short-range radio link, such as Bluetooth, IEEE 802.11, and/or a proprietary wireless protocol. The communications link may facilitate uni-directional and/or bidirectional communication between the IMD 102 and the receiving device 106. Data and/or control signals may be transmitted between the IMD 102 and the receiving device 106 to coordinate the functions of the IMD 102 and/or the receiving device 106. Patient data may be downloaded from one or more of the IMD 102 and the receiving device 106 periodically or on command. The physician and/or the patient may communicate with the IMD 102 and the receiving device 106, for example, to acquire patient data or to initiate, terminate, or modify recording and/or therapy.

The illustrative system 100 shown in FIG. 1 is not intended to suggest any limitation as to the scope of use or functionality of embodiments of the subject matter disclosed throughout this disclosure. Neither should the illustrative system 100 be interpreted as having any dependency or requirement related to any single component or combination of components illustrated in FIG. 1. For example, in embodiments, the illustrative system 100 may include additional components. Additionally, any one or more of the components depicted in FIG. 1 can be, in embodiments, integrated with various ones of the other components depicted therein (and/or components not illustrated). Any number of other components or combinations of components can be integrated with the illustrative system 100 depicted in FIG. 1, all of which are considered to be within the ambit of this disclosure.

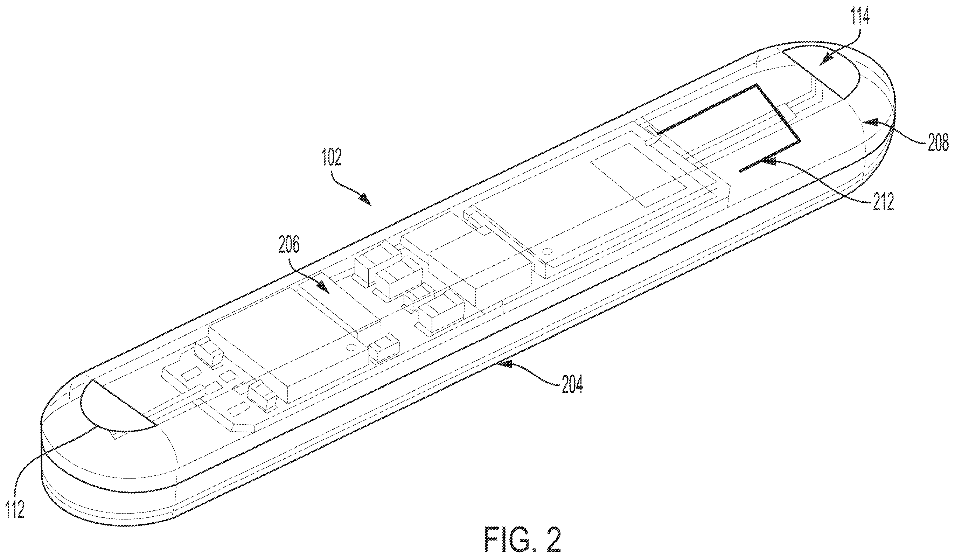

Fig. 2 is a perspective view of an IMD 102 including a power source housing 204, a printed circuit board 206, non-conductive enclosure 208, a first electrode 112, a second electrode 114, and an antenna 212.

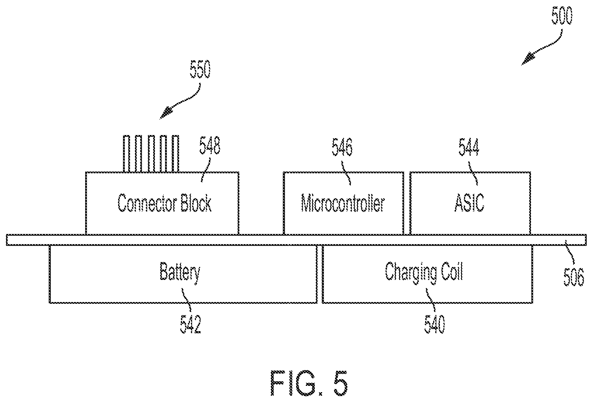

In some embodiments, the power source housing 204 is configured to enclose a power source (e.g., the battery depicted in FIG. 5). Additionally, or alternatively, the printed circuit board 206 may be arranged on a surface of the power source housing 204. According to embodiments, the surface of the power source housing 204 may be planar or approximately planar.

In some examples, the non-conductive enclosure 208 is molded over the power source housing 204 and the printed circuit board 206. In certain instances, the non-conductive enclosure 208 is configured to enclose the components and electrical interconnects of the IMD 102 in order to hermetically seal the components of the IMD 102. Various embodiments include the non-conductive material of the non-conductive enclosure 208 to be characterized by dielectric properties. In certain instances, this material is a liquid crystal polymer or an epoxy.

The use of a non-conducting material may reduce the need for an epoxy header typically used for transmitting signals, allowing for more space in the IMD 102 to be used for a larger power source housing 204, as explained in more detail below.

The first electrode 112 and second electrode 114 may be arranged on the outer surface of the non-conductive enclosure 208 and coupled to the printed circuit board 206 with connector traces. In various embodiments there may be two or more electrodes arranged on the outer surface of the non-conductive enclosure 208. In certain instances, there may be one or more external electrodes placed on the surface of the non-conductive housing that is above and/or is not above the printed circuit board 206.

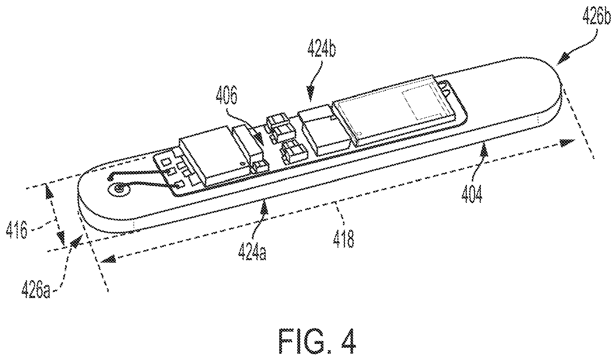

In various embodiments, the IMD 102 contains an antenna 212 arranged within or on the non-conductive housing 208 and is connected to the printed circuit board 206. In certain instances, the antenna 212 may include a first portion arranged parallel to a longitudinal axis of the housing 204, wherein the longitudinal axis extends from a first end (e.g., the first end 426a of FIG. 4) of the IMD 102 to a second end (e.g., the second end 426b of FIG 4) of the IMD 102. In certain instances, the antenna 212 may include a second portion coupled to the first portion, wherein the second portion is arranged perpendicular to the longitudinal axis of the housing. In additional examples, the antenna 212 may include a third portion coupled to the second portion, wherein the third portion is arranged parallel to the longitudinal axis of the housing.

In certain instances, the antenna 212 is positioned on or within the non-conductive enclosure 208 without being in contact with the conductive material of the power source housing 204, to maintain the proper function of the antenna 212. The positioning of the antenna 212 within or on the non-conductive enclosure 208, as opposed to in a header located at the end of the IMD 102, may reduce or eliminate the need for a header and/or increase the size of the power supply housed in the power supply housing 204 for the same size IMD 102. For example, the length of the power source may extend the length of the power source housing 204 whereas if the IMD 102 had a header the power supply could only extend partially the length of the power source housing 204. Thus, when there is no header, the power source can be of larger size without requiring a larger IMD 102.

In certain instances, the IMD 102 will be encased in a hermetic seal to provide a seal between the device and the surroundings. Further, this seal may be comprised of an atomic layer deposit.

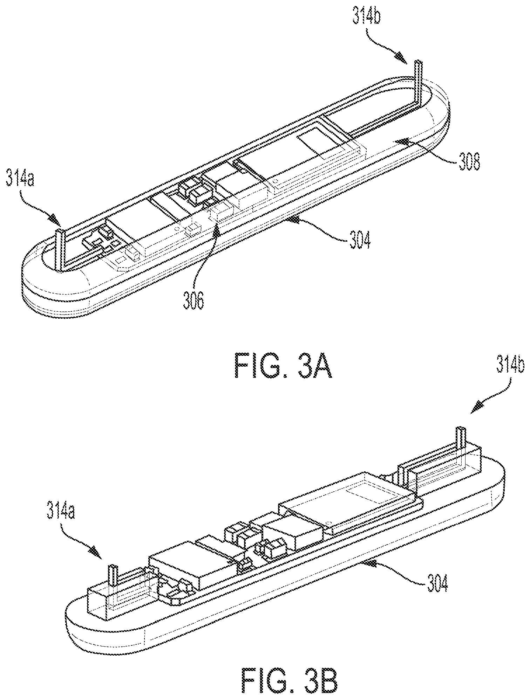

Fig. 3A is a perspective view of an implantable device 102, including the non-conductive enclosure 308, printed circuit board 306, power source housing 304, and connector traces 314a, 314b. In certain instances, the IMD 102 uses the connector traces 314a, 314b to connect the electrodes 112,114 to the printed circuit board 306.

Fig. 3B is a front-facing view of a printed circuit board 306 coupled to a power source housing 304, including frames 316a, 316b that guide and/or support the connector traces 314a, 314b. For example, the connector traces 314a, 314b are secured on the power source housing 306 by one or more frames 316a, 316b. In various embodiments, first segments of the traces 316a, 316b are connected to the printed circuit board and extend along the housing 306, e.g., along a longitudinal axis of the housing 304. In certain instances, a second segment of the traces can be arranged to project from the power source housing 306, as illustrated. According to some embodiments, the frames 316a, 316b are configured to create boundaries for the connector traces 314a, 314b as to reduce their movement within the medical device 102. In various embodiments, the frames 316a, 316b may be composed by a non-conductive material (e.g., a plastic material). While the illustrated embodiment of Fig. 3B contains two connector traces, two frames, and two electrodes, in certain instances, embodiments may contain more than two connector traces and more than two frames, and/or more than two electrodes.

Fig. 4 is a front facing image of components of an IMD 102 including a power source housing 404 and printed circuit board 406. In various embodiments the IMD 102 includes a power source housing 404, including a first side 424a and a second side 424b that extend along a longitudinal axis between a first end 426a and second end 426b. The first end 426a may be opposite the second end 426b and the distance between the first end 426a and second end 426b is greater than the distance between the first side 424a and the second side 424b. The first end 426a and second end 426b may have an equal dimension 416. The first side 424a and second side 424b may have an equal dimension 418. The size of the power source that is included within the power source housing 404 may cause the dimension lengths 416, 418 to increase or decrease. In certain instances, as mentioned previously, a larger power source is advantageous to the IMD 102. In these instances, a power source housing with larger dimension lengths 416, 418 may be desired. In certain instances, the power source housing 406 is comprised of a conductive material. In certain instances, this conductive material is a metal.

FIG. 5 shows an electrical subassembly 500 which may be detachably and electrically coupled with the printed circuit board 306. The subassembly 500 is disposed onto the power source housing 304 and includes a battery 544, a charging coil 540 for wireless charging of the battery 544 using an external charging device 652.

The subassembly 500 may also include one or more connector blocks 548 fixed such that contacts 550 within the connector blocks 548 make electrical contact with the traces 314 of the IMD 102 when the connector blocks 548 are attached to the printed circuit board 306. The number of contacts 550 may be the same as the number of traces 314 such that each of the contacts 550 makes a one-to-one connection with each of the traces 314.

The subassembly 500 may also include control circuitry such as a microcontroller 546, and one or more Application Specific Integrated Circuit (ASICs) 544 as suitable. ASIC(s) 544 may include current generation circuitry for providing stimulation pulses at one or more of the electrodes 112 and 114 and may also include telemetry modulation and demodulation circuitry for enabling bidirectional wireless communications at the antenna 212, battery charging and protection circuitry couplable to charging coil 540, DC-blocking capacitors in each of the current paths proceeding to the electrodes 112 and 114, etc. Components are integrated via a printed circuit board (PCB) 306.

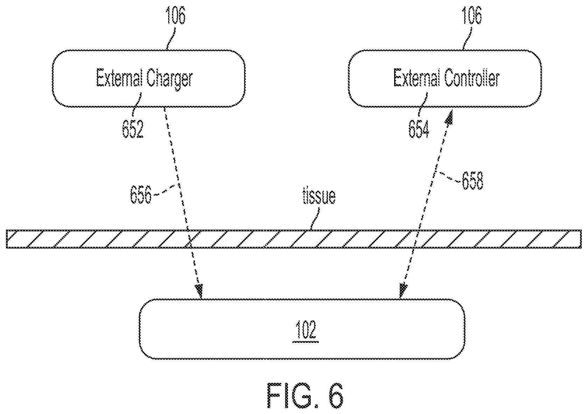

FIG. 6 further shows the external components (for example, the receiving device 106) referenced above, which may be used to communicate with the IMD 102. The receiving device 106 may include an external charger 652 and an external controller 346. The external controller 654 may be used to control and monitor the IMD 102 via a bidirectional wireless communication link 658 passing through a patient's tissue. For example, the external controller 654 may be used to monitor the measurements taken by the electrodes 112 and 114.

Communication on the wireless communication link 658 can occur via magnetic inductive coupling between an antenna (not shown) in the external controller 654 and the antenna 212 in the IMD 102. The magnetic field comprising the link 658 may be modulated via Frequency Shift Keying (FSK) or the like, to encode transmitted data. Other methods including but not limited to short-range RF telemetry (e.g., Bluetooth, WiFi, Zigbee, MICS, etc.) may also be employed.

The external charger 652 can provide power to recharge the battery 542 when the battery 542 is rechargeable. Such power transfer may occur by energizing a charging coil (not shown) in the external charger 552, which produces a magnetic field 656 which then energizes the charging coil 540 in the subassembly 500, which is rectified, filtered, and used to recharge the battery 542.

Furthermore, the antenna 212 may be positioned to face the tissue or positioned to be at the location closest to the skin side or the exterior side of the patient's body, in order to minimize or avoid RF interference by having less body tissue to transmit wireless data therethrough. In addition, the integrated circuitry in some examples includes a Kelvin connection to the first electrode 110 and the second electrode 114. In certain instances, the subassembly 500 may include an accelerometer to determine whether or not the IMD 102 has turned or flipped. The accelerometer may determine periods of electrode inactivity to determine a stable signal and select between the first electrode 112 and the second electrode 114.

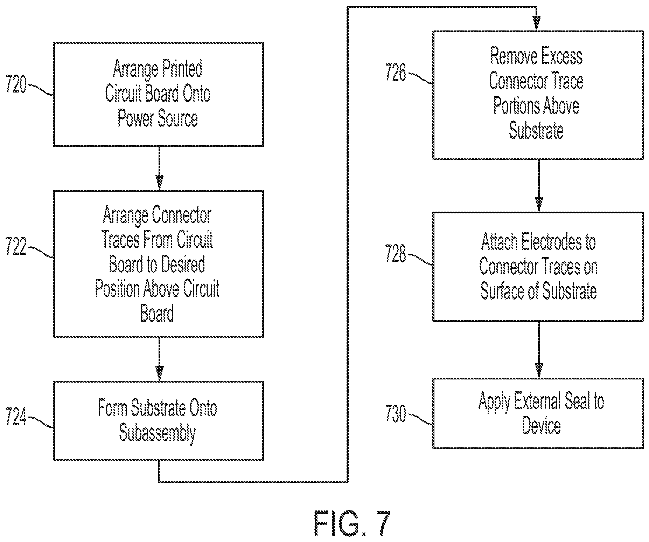

Fig. 7 is a flow chart of a method for forming a hermetically-sealed IMD 102. In various embodiments, the IMD 102 may be formed by arranging a printed circuit board subassembly onto a power source housing 720. Then, the connector traces may be arranged from the circuit board to the desired position above the circuit board 722. In certain instances, the first segments of connector traces are arranged along the housing and the second segments of the connector traces are arranged to project from the housing. In these examples, the first ends of the connector traces connect to the circuit board assembly. In certain instances, the first ends of the connector traces are connected to the circuit board assembly by soldering the components together. In certain embodiments, one or more frames are arranged over the first segments of the connector traces to hold the first segments of the connector traces in place on the power source housing and printed circuit board subassembly.

The non-conductive enclosure may then be formed over the subassembly 724, leaving portions of the second segment of the connector traces exposed. In certain instances, this is followed by the removal of portions of the second segments of the connector trace exposed above the outer surface 726. In various embodiments, this is step may be followed by the attaching of electrodes to the connector traces on the surface of the non-conductive enclosure 728. In certain instances, the electrodes can be attached to the connector traces on the surface through the methods of a laser weld, deposit, sputter or a spray/ink jet method. In various embodiments, a seal can be applied to the entire device 730 to hermetically seal the device, after masking the electrodes. In certain instances, this seal is an atomic layer deposit. In certain instances, an antenna may be formed within or on the non-conductive enclosure.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features.
The invention is set out in the claims that follow:

## Claims

1. An implantable medical device, IMD (102), configured to sense one or more physiological parameters of a subject, the IMD (102) comprising:
a power source;
a housing (110, 204) comprising a conductive material and enclosing the power source, the housing (102) comprising a first side, a second side, a first end, and a second end, wherein the first side is opposite the second side and the first end is opposite the second end, and wherein a first distance between the first and second ends is greater than a second distance between the first and second sides;
a printed circuit board (206) arranged on the first side of the housing (110, 204) and conductively coupled to the power source;
a non-conductive enclosure (208) arranged over the printed circuit board (206) and hermetically sealing the printed circuit board, the non-conductive enclosure comprising an outer surface; and
first and second electrodes (112, 114) arranged on the outer surface of the non-conductive enclosure (208), wherein the first external electrode (112) is coupled to the printed circuit board (206) by a first trace and the second external electrode (114) is coupled to the printed circuit board by a second trace.

2. The IMD (102) of claim 1, wherein the non-conductive enclosure (208) is formed from a liquid crystal polymer or an epoxy.

3. The IMD (102) of any one of claims 1-2, wherein the connector traces are secured in place on the housing (110) by one of more frames.

4. The IMD (102) of claim 3, wherein the frames are composed of non-conductive material and create boundaries to limit the movement of the traces.

5. The IMD (102) of any one of claims 1-4, wherein the device further comprises an antenna (212) arranged within or on the non-conductive enclosure (208) and coupled to the circuit board (206).

6. The IMD (102) of claim 5, wherein the antenna (212) comprises a first portion arranged parallel to the longitudinal axis.

7. The IMD (102) of claim 6, wherein the antenna further comprises a second portion arranged perpendicular to the longitudinal axis.

8. The IMD (102) of any one of claims 1-7, wherein the outer surface of the IMD (102) comprises an external seal configured to hermetically seal the IMD (102) from the surroundings.

9. The IMD (102) of claim 8, wherein the external seal is an atomic deposit layer.

10. The IMD (102) of any one of claims 1-9, wherein the conductive material is a metallic material.

11. The IMD (102) of any one of claims 1-10, further comprising a third electrode and a fourth electrode arranged on an outer surface of the housing.

12. A method of forming a hermetically-sealed implantable device (102) comprising
arranging a circuit board subassembly onto a housing (110, 204) enclosing a power source, the housing (110, 204) comprising a conductive material;
arranging first segments of connector traces along the housing and arranging second segments of the connector traces to project from the housing (110, 204), wherein first ends of the connector traces connects to the circuit board subassembly;
arranging one or more frames over the first segments of the connector traces to hold the first segments of the connector traces in place;
forming a non-conductive enclosure (208) over the subassembly, to create an encasing that leaves portions of the second segments of the connector traces exposed above the outer surface of the non-conductive enclosure;
removing the portions of the second segments of the connector traces exposed above the outer surface; and
arranging two electrodes (112, 114) on the outer surface of the non-conductive enclosure (208) and connecting the two electrodes (112, 114) to the connector traces.

13. The method of claim 12, wherein the electrodes (112, 114) are arranged on other surface of the non-conductive enclosure (208) through one of the techniques of a laser weld, deposit, sputter, or spray/ink jet method.

14. The method of claim 13, wherein a hermetic seal is applied to the implantable device, after arranging the electrodes (112, 114) on the outer surface.

15. The method of any one of claims 12-14, further comprising forming an antenna (212) within or on the non-conductive enclosure (208).

## Patentansprüche

1. Implantierbare medizinische Vorrichtung, IMD (102), die eingerichtet ist, einen oder mehrere physiologische Parameter eines Subjekts zu erfassen, wobei die IMD (102) umfasst:
eine Energiequelle;
ein Gehäuse (110, 204), das ein leitendes Material umfasst und die Energiequelle umschließt, wobei das Gehäuse (102) eine erste Seite, eine zweite Seite, ein erstes Ende und ein zweites Ende umfasst, wobei die erste Seite der zweiten Seite gegenüberliegt und das erste Ende dem zweiten Ende gegenüberliegt, und wobei ein erster Abstand zwischen dem ersten und dem zweiten Ende größer ist als ein zweiter Abstand zwischen der ersten und der zweiten Seite;
eine Leiterplatte (206), die auf der ersten Seite des Gehäuses (110, 204) angeordnet und leitend mit der Energiequelle verbunden ist;
eine nichtleitende Umhüllung (208), die über der Leiterplatte (206) angeordnet ist und die Leiterplatte hermetisch abdichtet, wobei die nichtleitende Umhüllung eine Außenfläche aufweist; und
eine erste und eine zweite Elektrode (112, 114), die auf der Außenfläche der nichtleitenden Umhüllung (208) angeordnet sind, wobei die erste externe Elektrode (112) über eine erste Leiterbahn mit der Leiterplatte (206) verbunden ist und die zweite externe Elektrode (114) über eine zweite Leiterbahn mit der Leiterplatte verbunden ist.

2. IMD (102) nach Anspruch 1, wobei die nichtleitende Umhüllung (208) aus einem Flüssigkristallpolymer oder einem Epoxid gebildet ist.

3. IMD (102) nach einem der Ansprüche 1-2, wobei die Verbinder-Leiterbahnen durch einen oder mehrere Rahmen am Gehäuse (110) befestigt sind.

4. IMD (102) nach Anspruch 3, wobei die Rahmen aus nichtleitendem Material bestehen und Begrenzungen bilden, um die Bewegung der Leiterbahnen zu begrenzen.

5. IMD (102) nach einem der Ansprüche 1-4, wobei die Vorrichtung ferner eine Antenne (212) umfasst, die innerhalb oder auf der nichtleitenden Umhüllung (208) angeordnet und mit der Leiterplatte (206) gekoppelt ist.

6. IMD (102) nach Anspruch 5, wobei die Antenne (212) einen ersten Teil umfasst, der parallel zur Längsachse angeordnet ist.

7. IMD (102) nach Anspruch 6, wobei die Antenne ferner einen zweiten Teil umfasst, der senkrecht zur Längsachse angeordnet ist.

8. IMD (102) nach einem der Ansprüche 1-7, wobei die Außenfläche der IMD (102) eine äußere Dichtung umfasst, die eingerichtet ist, die IMD (102) hermetisch gegenüber der Umgebung abzudichten.

9. IMD (102) nach Anspruch 8, wobei die äußere Abdichtung eine atomare Ablagerungsschicht ist.

10. IMD (102) nach einem der Ansprüche 1-9, wobei das leitende Material ein metallisches Material ist.

11. IMD (102) nach einem der Ansprüche 1-10, ferner eine dritte Elektrode und eine vierte Elektrode umfassend, die an einer Außenfläche des Gehäuses angeordnet sind.

12. Verfahren zur Herstellung einer hermetisch abgedichteten implantierbaren Vorrichtung (102), umfassend
Anordnen einer Leiterplatten-Unteranordnung auf einem Gehäuse (110, 204), das eine Energiequelle umschließt, wobei das Gehäuse (110, 204) ein leitendes Material umfasst;
Anordnen erster Segmente von Verbinder-Leiterbahnen entlang des Gehäuses und Anordnen zweiter Segmente der Verbinder-Leiterbahnen, so dass sie aus dem Gehäuse (110, 204) herausragen, wobei erste Enden der Verbinder-Leiterbahnen mit der Leiterplattenunteranordnung verbunden sind;
Anordnen eines oder mehrerer Rahmen über den ersten Segmenten der Verbinder-Leiterbahnen, um die ersten Segmente der Verbinder-Leiterbahnen an ihrem Platz zu halten;
Ausbilden einer nichtleitenden Umhüllung (208) über der Unteranordnung, um eine Ummantelung zu schaffen, die Teile der zweiten Segmente der Verbinder-Leiterbahnen oberhalb der Außenfläche der nichtleitenden Umhüllung freilässt;
Entfernen der Teile der zweiten Segmente der Verbinder-Leiterbahnen, die oberhalb der Außenfläche freiliegen; und
Anordnen von zwei Elektroden (112, 114) auf der Außenfläche der nichtleitenden Umhüllung (208) und Verbinden der beiden Elektroden (112, 114) mit den Verbinder-Leiterbahnen.

13. Verfahren nach Anspruch 12, wobei die Elektroden (112, 114) auf einer anderen Oberfläche des nichtleitenden Gehäuses (208) durch eine der Techniken Laserschweißen, Auftragen, Sputtern oder Spritzen/Tintenstrahlverfahren angeordnet werden.

14. Verfahren nach Anspruch 13, wobei eine hermetische Abdichtung auf die implantierbare Vorrichtung aufgebracht wird, nachdem die Elektroden (112, 114) auf der äußeren Oberfläche angeordnet wurden.

15. Verfahren nach einem der Ansprüche 12 bis 14, ferner Ausbilden einer Antenne (212) innerhalb oder auf der nichtleitenden Umhüllung (208) umfassend.

## Revendications

1. Dispositif médical implantable, IMD, (102) configuré pour détecter un ou plusieurs paramètres physiologiques d'un sujet, l'IMD (102) comprenant :
une source d'alimentation ;
un boîtier (110, 204) comprenant un matériau conducteur et renfermant la source d'alimentation, le boîtier (102) comprenant un premier côté, un second côté, une première extrémité et une seconde extrémité, dans lequel le premier côté est opposé au second côté et la première extrémité est opposée à la seconde extrémité et dans lequel une première distance entre les première et seconde extrémités est plus grande qu'une seconde distance entre les premier et second côtés ;
une carte de circuit imprimé (206) agencée sur le premier côté du boîtier (110, 204) et couplée par conduction à la source d'alimentation ;
une enceinte non conductrice (208) agencée au-dessus de la carte de circuit imprimé (206) et scellant hermétiquement la carte de circuit imprimé, l'enceinte non conductrice comprenant une surface externe ; et
des première et deuxième électrodes (112, 114) agencées sur la surface externe de l'enceinte non conductrice (208), dans lequel la première électrode externe (112) est couplée à la carte de circuit imprimé (206) par une première piste et la deuxième électrode externe (114) est couplée à la carte de circuit imprimé par une seconde piste.

2. IMD (102) selon la revendication 1, dans lequel l'enceinte non conductrice (208) est formée à partir d'un polymère à cristaux liquides ou d'un époxy.

3. IMD (102) selon l'une quelconque des revendications 1 et 2, dans lequel les pistes de connecteur sont fixées en place sur le boîtier (110) au moyen d'un ou de plusieurs moyens de restriction.

4. IMD (102) selon la revendication 3, dans lequel les moyens de restriction sont constitués à partir d'un matériau non conducteur et ils créent des frontières pour limiter le déplacement des pistes.

5. IMD (102) selon l'une quelconque des revendications 1 à 4, dans lequel le dispositif comprend en outre une antenne (212) agencée à l'intérieur de l'enceinte non conductrice (208) ou sur celle-ci et couplée à la carte de circuit imprimé (206).

6. IMD (102) selon la revendication 5, dans lequel l'antenne (212) comprend une première partie agencée parallèlement à l'axe longitudinal.

7. IMD (102) selon la revendication 6, dans lequel l'antenne comprend en outre une seconde partie agencée perpendiculairement à l'axe longitudinal.

8. IMD (102) selon l'une quelconque des revendications 1 à 7, dans lequel la surface externe de l'IMD (102) comprend un scellement externe configuré pour sceller hermétiquement l'IMD (102) vis-à-vis de son environnement.

9. IMD (102) selon la revendication 8, dans lequel le scellement externe est une couche de dépôt atomique.

10. IMD (102) selon l'une quelconque des revendications 1 à 9, dans lequel le matériau conducteur est un matériau métallique.

11. IMD (102) selon l'une quelconque des revendications 1 à 10, comprenant en outre une troisième électrode et une quatrième électrode agencées sur une surface externe du boîtier.

12. Procédé de formation d'un dispositif implantable scellé hermétiquement (102) comprenant :
l'agencement d'un sous-assemblage de carte(s) de circuit sur un boîtier (110, 204) qui renferme une source d'alimentation, le boîtier (110, 204) comprenant un matériau conducteur;
l'agencement de premiers segments de pistes de connecteur le long du boîtier et l'agencement de seconds segments des pistes de connecteur de telle sorte qu'ils fassent saillie depuis le boîtier (110, 204), dans lequel des premières extrémités des pistes de connecteur sont connectées au-sous assemblage de carte(s) de circuit ;
l'agencement d'un ou de plusieurs moyens de restriction au-dessus des premiers segments des pistes de connecteur afin de maintenir en place les premiers segments des pistes de connecteur;
la formation d'une enceinte non conductrice (208) au-dessus du sous-assemblage, afin de créer un enveloppement qui laisse des parties des seconds segments des pistes de connecteur exposées au-dessus de la surface externe de l'enceinte non conductrice ;
l'enlèvement des parties des seconds segments des pistes de connecteur exposées au-dessus de la surface externe ; et
l'agencement de deux électrodes (112, 114) sur la surface externe de l'enceinte non conductrice (208) et la connexion des deux électrodes (112, 114) sur les pistes de connecteur.

13. Procédé selon la revendication 12, dans lequel les électrodes (112, 114) sont agencées sur une autre surface de l'enceinte non conductrice (208) par l'intermédiaire de l'une des techniques comprenant un procédé de soudage au laser, un procédé de dépôt, un procédé de pulvérisation et un procédé de vaporisation/par jet d'encre.

14. Procédé selon la revendication 13, dans lequel un scellement hermétique est appliqué sur le dispositif implantable, après l'agencement des électrodes (112, 114) sur la surface externe.

15. Procédé selon l'une quelconque des revendications 12 à 14, comprenant en outre la formation d'une antenne (212) à l'intérieur de l'enceinte non conductrice (208) ou sur celle-ci.
